# EUROPEAN PATENT SPECIFICATION

(11) **EP 1 901 786 B1**
(45) Date of publication and mention of the grant of the patent: **01.12.2010**
(21) Application number: 06754491.6
(22) Date of filing: 22.06.2006
(51) Int. Cl.: A61P 41/00

(54) **BIOMATERIALS IN THE FORM OF FIBRES FOR USE AS MEDICAL DEVICES IN THE TREATMENT OF WOUNDS, AND THEIR PRODUCTION PROCESS**
BIOMATERIALIEN IN FORM VON FASERN ZUR VERWENDUNG ALS MEDIZINPRODUKT BEI DER BEHANDLUNG VON WUNDEN, UND IHR HERSTELLUNGSVERFAHREN
BIOMATERIAUX SOUS FORME DE FIBRES DESTINES A SERVIR DE DISPOSITIFS MEDICAUX POUR LE TRAITEMENT DE BLESSURES, ET LEUR PROCEDE DE PRODUCTION

(30) Priority: 07.07.2005 IT PD20050206
(43) Date of publication of application: 26.03.2008
(73) Proprietor: FIDIA ADVANCED BIOPOLYMERS S.R.L., 35031 Abano Terme (Padova) (IT)
(72) Inventor: BELLINI, Davide, I-35020 Albignasego (PD) (IT); TERRASSAN, Massimo, I-35020 Albignasego (PD) (IT); PAVESIO, Alessandra, I-35141 Padova (IT)
(74) Representative: Bianchetti, Giuseppe
(86) International application number: PCT/EP2006/005992
(87) International publication number: WO 2007/006403

(56) References cited:
- WO-A-93/11803
- WO-A-2006/037592
- WO-A2-02/18448
- WO-A2-99/04828
- US-A1- 2004 242 535
- US-B1- 6 339 074
- MO Y ET AL: "Rheological evidence of the gelation behavior of hyaluronan-gellan mixtures." BIORHEOLOGY 2000, vol. 37, no. 5-6, 2000, pages 401-408, XP009078949 ISSN: 0006-355X

## Description

### SUBJECT OF THE INVENTION

The present invention relates to new biomaterials in the form of fibres, essentially consisting of a mixture of gellan and hyaluronic acid derivatives, for use in surgery, especially in the treatment of wounds and mild burns. The present invention also relates to the process for the preparation of said biomaterials.

### BACKGROUND OF THE INVENTION

The treatment of wounds of mild to medium severity, be they abrasions, excoriations, mild burns or cuts, is performed by applying a vast range of medical devices which are intended to protect the lesion, enhance wound healing, avoid necrosis, absorb any exudate formed and possibly release biologically and/or pharmacologically active substances that help achieve complete healing. There are many devices currently on the market which can be subdivided according to their characteristics into
biocompatible, non-resorbable: for example polyurethane sponges (V.A.C^{®}, KCl Medical; Suprasorb^{®}, Lohmann & Rauscher) or self-adhesive silicon sheets (Cica-Care, Smith & Nephew);
biocompatible, partially resorbable: materials based on carboxymethylcellulose derivatives (Aquacel^{®}, Convatec);
biocompatible, completely resorbable: among others, the materials consisting of hyaluronic acid derivatives (Hyalofill^{®}, Fidia Advanced Biopolymers, the hyaluronic acid benzyl ester).

The device to be used in each case is selected according to individual requirements; in the case of extremely exudative wounds that need several dressing changes in the course of 24 hours, a biocompatible but not biodegradable device that can be easily removed without causing bleeding or loss of tissue during dressing changes is suitable. In the case of wounds of mild to medium severity and/or mild burns, which are dealt with by the present invention, biocompatible and bioresorbable dressings are preferable (because they need not be removed). However, they are limited by being degraded somewhat rapidly and therefore remaining only briefly at the application site. They therefore need renewing several times before the wound has healed and are not very functional in terms of efficacy, practicality, patient compliance and costs.

These limitations can be overcome by the biomaterials of the present invention, which are in the form of fibres consisting of an association of gellan and hyaluronic acid derivatives, characterised by suitable bioadhesiveness, elasticity, absorbency, biocompatibility and biodegradability/bioresorbability.

The biomaterials of the present invention remain *in situ* for long enough to allow the underlying tissues to heal, they absorb and retain exudate from the would and prevent dehydration; these dressings are easy to handle, they can be adapted to fit wounds of any size or shape and are flexible and therefore comfortable to wear. They are made by a simple, easy to perform process, that translates into a marked reduction in industrial costs.

Associations between gellan and hyaluronic acid and/or the derivatives thereof are already known to the state of the art. For example, US patent application No. 2004/0242535 describes one such association in liquid form which gives a compact and very viscous gel that does not slip off the wound, once applied by spraying or spreading on exuding skin lesions.

However, the present invention differs substantially from the state of the art in that:
1. the two polymers are co-extruded together and form a solid matrix, that is compact and can be moulded to shape, it is not a gel to be sprayed and/or spread;
2. the fibres are always composed of over 80% gellan;
3. the absorbent potential of the matrix is far greater than that of the gel, because the fibres are made with absolutely unhydrated gellan, unlike the gel, which needs to be pre-hydrated.

Therefore, the dressings of the invention, consisting of a mixture of gellan and hyaluronic acid derivatives, represent a decisive advance over the state of the art in the field of biocompatible/bioresorbable dressings for exuding skin wounds of mild to medium severity.

Hyaluronic acid (HA) is a molecule that has been known for some time. It is a heteropolysaccharide composed of alternating residues of D-glucuronic acid and N-acetyl-D-glucosamine, and has a linear chain and a molecular weight ranging between 50,000 and 13 x 10⁶ Da, according to the source from which it is extracted and/or the method used to prepare it. It is present in nature in the pericellular gels, in the fundamental substance of the connective tissue of vertebrate organisms (of which it represents one of the main components), in the synovial fluid of joints, the vitreous humor and umbilical cord.

HA is therefore of fundamental importance in the biological organism, especially as a support for the cells of many tissues such as the skin, tendons, muscles and cartilage.

Hyaluronic acid modulates, through its CD44 receptor membrane, many different processes relative to cell physiology and biology, such as cell proliferation, migration and differentiation and angiogenesis, and has other functions too, such as tissue hydration and joint lubrication. Moreover, it has been demonstrated that HA plays a fundamental role in the tissue repair process, both from a structural point of view (in organising the extracellular matrix and regulating its hydration) and in stimulating a vast range of processes in which it intervenes both directly and indirectly (clot formation, phagocyte activity, fibroblast proliferation, neovascularisation, reepithelialisation, etc.) (Weigel P. et al., J Theoretical Biol, 1986:219-234; Abatangelo G. et al., J Surg Res, 1983, 35:410-416; Goa K. et al., Drugs, 1994, 47:536-566). These well-known properties have long been put to use in preparing dressings for wounds, ulcers and skin lesions of various origin.

Hyaluronic acid can be obtained from any source, for example, it can be obtained by extraction from rooster combs (EP 138572 B1), by fermentation (EP 716688 B1), or by technological means, and its molecular weight may vary between 400 and 3x10⁶Da.

Hyaluronic acid has been chemically modified in various ways, giving polymers that keep the biological/pharmacological characteristics of the starting molecule, but are easier to process and give a better mechanical performance. Particularly suitable for the purposes of the present invention are the hyaluronic acid derivatives obtained by:
esterification with alcohols of the aliphatic, araliphatic, cycloaliphatic, aromatic, cyclic and heterocyclic series, with a percentage of esterification that may vary according to the type and length of the alcohol used, between 0.1 and 100% while any remaining percentage of unesterified HA can be salified with organic and/or inorganic bases (HYAFF^{®} - EP 216453 B1). Excellent absorbent properties have also been attributed to these materials (EP 999859 B1);
inner esterification, with esterification not exceeding 20%, preferably between 0.05 and 10%, while the remaining, unesterified HA may be salified with organic and/or inorganic bases (ACP^{®} - EP 341745 B1);
amidation with amines of the aliphatic, araliphatic, cycloaliphatic, aromatic, cyclic and heterocyclic series, with amidation ranging between 0.1 and 50%, while the remaining HA that has not undergone amidation may be salified with organic and/or inorganic bases (HYADD™ - EP 1095064 B1);
O-sulphation to the 4^{th} degree of sulphation (EP 702699 B1).

Hyaluronic acid derivatives are already known for their ability to form fibres with which non-woven felts can be made for applications in various fields, including topical/dermatological applications. Indeed, patent No. EP 618817 B1 describes in detail the process by which it is possible to extrude, by wet or dry extrusion, a paste consisting of hyaluronic acid derivatives, especially ester derivatives, sometimes in association with other polymers including gellan, giving a mass of multifilaments. The multifilaments must be further treated to "fix" the fibres together at the points of contact, to give a three-dimensional structure composed of a mesh of tangled fibres known as a "non-woven" felt. The treatments to which such multifilaments are exposed are chiefly mechanical (carding and subsequent perforation with needles) and/or chemical (spraying the mass of fibres with binding agents that produce chemical coagulation) and they are indispensable for the purposes of obtaining a non-woven fabric; the material obtained can be used for example to prepare topical, absorbent, multilayer dressings (EP 683279 B1).

The object of present invention differs markedly from the state of the art because, in that according to the novel process described hereafter, the mass of fibres obtained by dry extrusion of a mixture of hyaluronic acid derivatives and gellan immediately gives rise to a three-dimensional matrix, without requiring any mechanical- and/or chemical-type intervention. The tangled mass of fibres thus obtained can still be defined as a non-woven fabric, but is new and different from those known to date, because it does not undergo any further treatment following extrusion apart from simple drying. The process of the invention is also very simple, easy to apply and economical on an industrial scale.

The hyaluronic acid derivatives listed above are associated with gellan at suitable, specific percentages, as has already been said.

Gellan is a hexopolysaccharide of microbial origin, produced from the micro-organism *Sphingomonas elodea* by aerobic fermentation.

Native gellan is a heteroglycan resulting from the linking of repetitive tetrasaccharide units, consisting of glucose, glucuronic acid and rhamnose in a molar ratio of 2:1:1; one of the glucose units is partially acetylated with O(6)-Acetyl and O(2)-L-Glyceryl groups. Deacetylation by alkaline hydrolysis gives the commercial product Gelrite^{®} (Fig. 1).

In solution, gellan has a structure formed by two parallel chains that wind round in a double helix stabilised by hydrogen bonds established between a methoxyl of one chain and a carboxyl of the other.

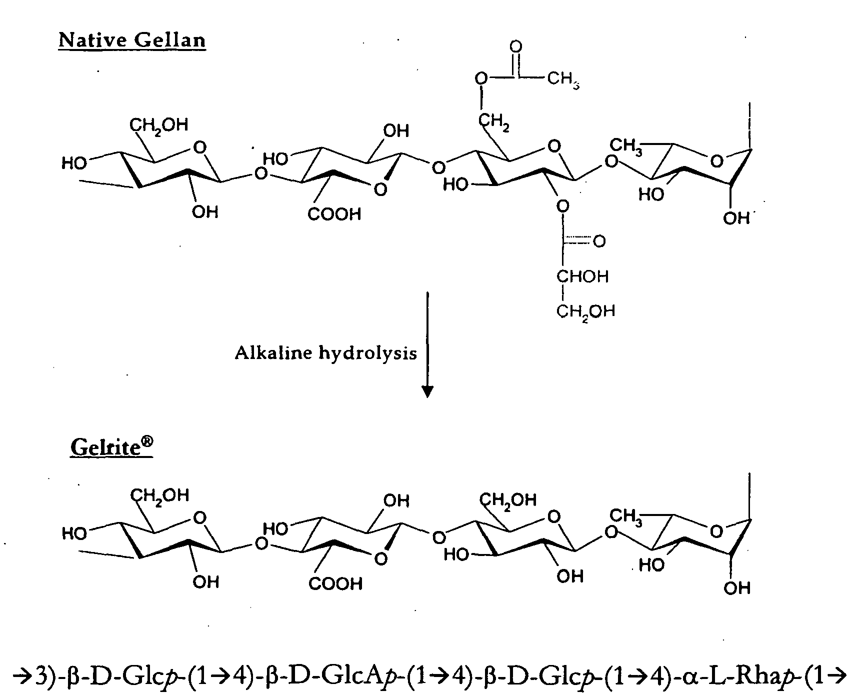

**Figure 1:** Primary structure of gellan and its deacetylated form. Gellan is capable of forming a gel in the presence of cations and at a suitable temperature: more precisely, native gellan forms elastic, flexible, but weak, gels, while the deacetylated derivative gives compact but fragile gels. The latter can be markedly improved by working with high concentrations of polymer and an adequate ionic strength. The gellan gels are biocompatible, biodegrade slowly and are highly absorbent.

Thanks to its characteristics, gellan is used in the pharmaceutical industry for the preparation of controlled-release microcapsules, eye drops that form gels once applied because of an interaction with the sodium ions physiologically present in the ocular fluid. Gellan is widely used in the vegetal biotechnology industry as a substitute in culture mediums for the more costly agarose, and in the food industry as a thickener and stabiliser.

Gellan is therefore used in various fields on account of its safety profile. Indeed it is defined as GRAS (Generally Regarded As Safe) by the U.S. Regulatory Body, the Food and Drug Administration.

The dry extrusion process of the present invention gives rise to fibres and subsequently three-dimensional matrices from which new medical devices can be made, that are biocompatible, biodegradable over a period of time that is compatible with the total healing of the lesion, highly absorbent, elastic, flexible, bioadhesive, adaptable with regard to shape and size to fit wounds of mild to medium severity, to which they are applicable, and they are able to facilitate wound healing. The simplicity of the production process keeps industrial costs relatively low. Consequently, the present invention is a remarkable improvement over the state of the art in the field of bioresorbable and biocompatible dressings.

### DETAILED DESCRIPTION OF THE INVENTION

The present invention relates to novel biomaterials in the form of fibres that can be made into a three-dimensional matrix, and the process for producing them. Said biomaterials are suitable for preparing medical devices to be used singly or in association with others to treat wounds of mild to medium severity (for example abrasions and deep excoriations, cuts) and mild burns. They can also be applied as absorbent tampons in surgery. The biomaterials of the invention are completely biocompatible and biodegradable (they do not therefore need to be removed from the application site), bioadhesive, elastic, and flexible, adaptable to the shape of the lesion, highly absorbent and able to facilitate the physiological healing process. They also remain at the site application for long enough to allow the wound to heal completely.

These characteristics are due to the polymers that constitute the new biomaterials, that is, gellan and a hyaluronic acid derivative, mixed in set percentages, and to the innovative process for preparing them, which will be described in detail hereafter.

As already mentioned, gellan is able to form a gel in the presence of cations and at certain temperatures. Commercial deacetylated gellan is suitable for the purpose because at high concentrations, at adequate ionic force and at the right temperature, it forms compact, elastic and flexible gels that are slow to biodegrade and are highly absorbent.

Hyaluronic acid is known to have been chemically modified in various ways to obtain polymers that maintain the biological/pharmacological characteristics of the starting molecule, but with better mechanical properties (for example, resistance, easier processing and handling) and adaptable biodegradability. Of the numerous derivatives, particularly useful for the purposes of the present invention, because of their high level of hydratability, are those obtained by
- esterification with alcohols of various kinds, particularly benzyl alcohol (HYAFF^{®} - 11), with a percentage of esterification that may vary between 0.1 and 100%, preferably between 50 and 75%;
- inner esterification, with a percentage of esterification not exceeding 20%, preferably between 0.05 and 10% (ACP^{®});
- amidation with amines of the aliphatic, araliphatic, cycloaliphatic, aromatic, cyclic and heterocyclic series with a percentage of amidation between 0.1 and 50% (HYADD™);
- O-sulphation of hyaluronic acid up to the 4th degree of sulphation.

Hydratability of the hyaluronic acid derivative is of fundamental importance to the extrusion process, as will be explained in detail hereafter, to maintain a moist environment that favours wound healing and to support the action of gellan in absorbing exudate, so as to avoid infections, necrosis of the newly formed tissue and abnormal scarring. Hyaluronic acid also exerts its biological/pharmacological action by intervening on diverse fronts. Indeed, it is involved in organising the extracellular matrix and regulating its hydration, and stimulates a vast series of processes in which it intervenes both directly and indirectly (clot formation, phagocyte activity, fibroblast proliferation, neovascularisation, reepithelialisation). Its prolonged presence *in situ* due to the slow biodegradability of gellan enhances these concerted effects.

The mixture of the two polymers at set concentration ratios enables the biomaterial to be prepared by a simple, effective, efficient process that can easily be applied on an industrial scale, and the characteristics of the three-dimensional matrices that derive therefrom are new and innovative compared to the state of the art.

The process used in the present invention involves dry extrusion of a mixture of polymers in suitable conditions of pressure and temperature and is innovative because the mass of filaments that derive from the extrusion phase form a compact, elastic and flexible matrix that can easily be cut to size, simply by drying it; no further intervention is therefore necessary, either of a mechanical or chemical nature. Such simplification facilitates manufacture and considerably limits industrial costs.

The process of the invention is carried out in two separate steps:

### 1 - preparation of the mixture of the two polymers

Powdered gellan and a hyaluronic acid derivative at a percentage of 80 and 20% respectively, preferably 90 and 10% and more preferably still 95 and 5% are mixed together at room temperature in dry form. The gellan used is one of the commercially available products, for example Kelcogel^{®} CG-LA, while the HA derivative can be an inner ester (ACP^{®}), an O-sulphated derivative, an amide derivative (HYADD™), an ester, preferably a benzyl ester with varying degrees of esterification, for example, 50% (HYAFF^{®} - 11p50) or 75% (HYAFF^{®} - 11p75). The mixture of powders is slowly supplemented with water until a concentration of between 80 and 120 mg/ml is achieved. The purpose of adding water is to hydrate the hyaluronic acid derivative, to form a paste that incorporates the gellan powder, known to be poorly water soluble at room temperature, thus making its extrusion possible. Thus, the gellan is actually extruded in non-hydrated form.

### 2 - extrusion

The mixture is placed in a screw extruder connected to a threading die with a diameter of between 30 and 300 µ. Extrusion is performed at specific pressure values and at temperature settings of between 45° and 65°C. The temperature setting is very important; indeed, because of gellan's thermoreversible properties, it melts at the aforesaid temperatures, so that the fibres obtained are compact, elastic and homogeneous, even though the original polymer paste was dishomogeneous in texture.

The fibres thus obtained are scattered on a conveyor where they rapidly set and, without any further intervention, form a compact, three-dimensional matrix that can be cut to the desired shape and size.

The described process gives fibres with a diameter that varies between 10 and 100 µ, while the matrix may measure between 25 and 200 cm² with a thickness of between 2 and 10 mm.

The biomaterial can be sterilised during packaging by the usual methods (for example, γ ray).

In view of the simplicity of the production technique and the favourable working conditions, the starting polymers can be supplemented with heat-stable pharmacologically and/or biologically active substances, for example metal ions with an antibacterial action, to give the fibres, and thus the final matrix, an actual pharmacological effect. Moreover, the matrices can be given further flexibility by co-extruding the polymer mixture with suitable plastifiers.

The present invention is further illustrated by the following examples.

### Example 1. Preparation of fibres composed of a mixture of gellan (98%) and HYAFF^{®} -11 p75 (2%)

9.8 grams of gellan Kelcogel^{®} CG-LA is dry-mixed with 0.2 grams of HYAFF^{®} -11 p75 in powder form.

The mixture of powders is placed in a mixer and slowly supplemented with 125 ml of water. The resulting paste has a concentration of 80 mg/ml. The paste is put in a screw extruder connected with a threading die of 60 µ. The temperature in the extrusion chamber is set at 54°C, and the material is extruded.

The resulting fibre has a mean diameter of 25-30 µ.

The final matrix is 2 mm thick. It is cut into pieces of 10X10 cm and sterilised by γ ray.

### Example 2. Preparation of fibres composed of a mixture of gellan (95% and HYAFF^{®} -11p50 (5%)

9.5 grams of gellan Kelcogel^{®} CG-LA are dry-mixed with 0.5 grams of HYAFF^{®} -11 p50 in powder form.

The mixture of powders is placed in a mixer and slowly supplemented with 110 ml of water. The concentration of the resulting paste is about 90 mg/ml. The paste is put in a screw extruder connected with a threading die of 40 µ. The temperature in the extrusion chamber is set at 54°C and the material is extruded.

The resulting fibre has a mean diameter of 15-25 µ.

The resulting matrix is 2 mm thick. It is cut into pieces of 10X10 cm and sterilised by γ ray.

### Example 3. Preparation of fibres composed of a mixture of gellan (90%) and sulphated HA with a degree of sulphation of 1 (10%)

9 grams of gellan Kelcogel^{®} CG-LA are dry-mixed with 1 gram of sulphated HA with a degree of sulphation of 1, freeze-dried.

The two polymers are placed in a mixer and slowly supplemented with 100 ml of water. The concentration of the resulting paste is 100 mg/ml. The paste is put in a screw extruder connected with a threading die of 150 µ. The temperature in the extrusion chamber is set at 54°C and the material is extruded.

The resulting fibre has a mean diameter of 50-70 µ.

The resulting matrix is 5 mm thick. It is cut into pieces of 5X5 cm and sterilised by γ ray.

### Example 4. Preparation of fibres composed of a mixture of gellan (95%) and HYADD™ (5%)

9.5 grams of gellan Kelcogel^{®} CG-LA are dry-mixed with 0.5 grams of HYADD™- 4 (hexadecyl amide with 2% of amidation) in powder form.

The two polymers are placed in a mixer and slowly supplemented with 125 ml of water. The concentration of the resulting paste is 80 mg/ml. The paste is put in a screw extruder connected with a threading die of 60 µ. The temperature in the extrusion chamber is set at 54°C and the material is extruded.

The resulting fibre has a mean diameter of 25-30 µ.

The final matrix is 2 mm thick. It is cut into pieces of 5X5 cm and sterilised by γ ray.

### Example 5. Preparation of fibres composed of a mixture of gellan (95%) and ACP^{®} (5%)

9.5 grams of gellan Kelcogel^{®} CG-LA are dry-mixed with 0.5 grams of ACP^{®} (auto-cross-linked hyaluronic acid) in powder form.

The two polymers are put in a mixer and slowly supplemented with 110 ml of water. The resulting paste has a concentration of about 90 mg/ml. It is placed in a screw extruder connected to a threading die of 100 µ. The temperature in the extrusion chamber is set at 50°C and the material is extruded.

A fibre with a mean diameter of 30-50 µ is obtained.

The final matrix, 2 mm thick, is cut into pieces of 10X10 cm and sterilised by γ ray.

## Claims

1. A three dimensional biomaterial in the form of fibres consisting of a mixture of at least 80% gellan and a hyaluronic acid derivative selected from esters, inner esters, amide derivatives, O-sulphated derivatives, for the surgical treatment of wounds of mild to medium severity.

2. Biomaterial according to claim 1 wherein the hyaluronic acid derivative is the benzyl ester with a degree of esterification ranging between 0.1 and 75%.

3. Biomaterial according to claim 2 wherein the degree of esterification of the benzyl ester ranges between 50 and 75%.

4. Biomaterial according to the previous claims further containing biologically and/or pharmacologically active substances.

5. Process for the production of a biomaterial as claimed in claims 1 to 4, comprising the steps of: a) mixing at least 80% gellan and the HA derivative as defied in claim 1 as dry powders and adding water to form a paste; b) extruding the paste with a screw extruder at temperatures between 45° and 65°C.

6. Process according to claim 5 wherein in step a) water is slowly added until a concentration of between 80 and 120 mg/ml is achieved.

7. Process according to claim 5 or 6 wherein the extruder is connected with a threading die with holes measuring between 30 and 300 µ in diameter.

8. Process according to claim 5 wherein the hyaluronic acid derivative is selected from inner esters, amide derivatives, O-sulphated derivatives, esters.

9. Process according to claim 8 wherein the hyaluronic acid derivative is the benzyl ester with a degree of esterification ranging between 0.1 and 75%.

10. Process according to claim 9 wherein the degree of esterification of the benzyl ester is between 50 and 75%.

11. Process according to claim 8 wherein the polymers further contain biologically and/or pharmacologically active substances.

12. Biomaterial in the form of fibres consisting of a mixture of gellan and a hyaluronic acid derivative, optionally containing biologically and/or pharmacologically active substances, obtained according to claims 5-10.

13. Use of the biomaterial according to claims 1-4 and 12 for the manufacture of a medicament in the surgical treatment of wounds of mild to medium severity, such as cuts, deep excoriations and abrasions, mild burns.

14. Use of the biomaterial according to claims 1-4 and 12 for the manufacture of a medicament to absorb serum and/or biological fluids in the course of surgery.

## Patentansprüche

1. Dreidimensianales Biomaterial in Form von Fasern, welches aus einer Mischung von mindestens 80% Gellan und einem Hyaluronsäurederivat, das ausgewählt ist aus Estern, inneren Estern, Amidderivaten, O-sulfonierten Derivaten, besteht, für die chirurgische Behandlung von Wunden leichterer bis mittlerer Schwere.

2. Biomaterial nach Anspruch 1, wobei das Hyaluronsäurederivat der Benzylester mit einem Veresterungsgrad zwischen 0,1 und 75% ist.

3. Biomaterial nach Anspruch 2, wobei der Veresterungsgrad des Benzylesters zwischen 50 und 75% beträgt.

4. Biomaterial nach den vorangegangenen Ansprüchen, welches weiterhin biologisch und/oder pharmakologisch aktive Substanzen enthält.

5. Verfahren zur Herstellung eines Biomaterials nach den Ansprüchen 1 bis 4, umfassend die Schritte a) Mischen von mindestens 80% Gellan und dem Hyaluronsäurederivat wie in Anspruch 1 definiert als trockene Pulver und Zugabe von Wasser zur Ausbildung einer Paste; b) Extrudieren der Paste mit einem Schneckenextruder bei Temperaturen zwischen 45° und 65°C.

6. Verfahren nach Anspruch 5, wobei in Schritt a) das Wasser langsam zugegeben wird bis eine Konzentration zwischen 80 und 120 mg/ml erreicht ist.

7. Verfahren nach Anspruch 5 oder 6, wobei der Extruder mit einer Schneidbacke mit Löchern, deren Durchmesser zwischen 30 und 300 µ misst, verbunden ist.

8. Verfahren nach Anspruch 5, wobei das Hyaluronsäurederivat ausgewählt ist aus inneren Estern, Amidderivaten, O-sulpfonierten Derivaten, Estern.

9. Verfahren nach Anspruch 8, wobei das Hyaluronsäurederivat der Benzylester mit einem Veresterungsgrad von 0,1 bis 75% ist.

10. Verfahren nach Anspruch 9, wobei der Veresterungsgrad des Benzylesters zwischen 50 und 75% beträgt.

11. Verfahren nach Anspruch 8, wobei die Polymere weiterhin biologisch und/oder pharmakologisch aktive Substanzen enthalten.

12. Biomaterial in Form von Fasern, welche aus einer Mischung aus Gellan und einem Hyaluronsäurederivat besteht, welches gegebenenfalls biologisch und/oder pharmakologisch aktive Substanzen enthält, erhalten gemäß den Ansprüchen 5-10.

13. Verwendung des Biomaterials nach Anspruch 1-4 und 12 für die Herstellung eines Medikaments in der chirurgischen Behandlung von Wunden leichterer oder mittlerer Schwere, wie Schnitten, tiefen Abschürfungen und Abrasionen, leichten Verbrennungen.

14. Verwendung des Biomaterials nach Anspruch 1-4 und 12 für die Herstellung eines Medikaments zur Absorption von Serum und/oder biologischer Flüssigkeiten im Behandlungsverlauf.

## Revendications

1. Biomatériau tridimensionnel sous la forme de fibres consistant en un mélange d'au moins 80% de gellane et d'un dérivé d'acide hyaluronique choisi parmi des esters, des esters internes, des dérivés d'amide, des dérivés O-sulfatés, pour le traitement chirurgical de blessures de gravité légère à moyenne.

2. Biomatériau selon la revendication 1 dans lequel le dérivé d'acide hyaluronique est l'ester benzylique avec un degré d'estérification dans la plage entre (0,1 et 75%.

3. Biomatériau selon la revendication 2 dans lequel le degré d'estérification de l'ester benzylique est dans la plage entre 50 et 75%.

4. Biomatériau selon les revendications précédentes contenant en outre des substances biologiquement et/ou pharmacologiquement actives.

5. Procédé de production d'un biomatériau selon les revendications 1 à 4, comprenant les étapes de : a) mélangeage d'au moins 80% de gellane et du dérivé d'AH tel que défini dans la revendication 1 sous forme de poudres sèches et ajout d'eau pour former une pâte ; b) extrusion de la pâte avec une extrudeuse à vis à des températures entre 45°C et 65°C.

6. Procédé selon la revendication 5 dans lequel à l'étape a) l'eau est ajoutée lentement jusqu'à obtenir une concentration entre 80 et 120 mg/ml.

7. Procédé selon la revendication 5 ou 6 dans lequel l'extrudeuse est reliée à une filière ayant des trous mesurant entre 30 et 300 µ de diamètre.

8. Procédé selon la revendication 5 dans lequel le dérivé d'acide hyaluronique est choisi parmi des esters internes, des dérivés d'amide, des dérivés O-sulfatés, des esters.

9. Procédé selon la revendication 8 dans lequel le dérivé d'acide hyaluronique est l'ester benzylique avec un degré d'estérification dans la plage entre 0,1 et 75%.

10. Procédé selon la revendication 9 dans lequel le degré d'estérification de l'ester benzylique est entre 50 et 75%.

11. Procédé selon la revendication 8 dans lequel les polymères contiennent en outre des substances biologiquement et/ou pharmacologiquement actives.

12. Biomatériau sous la forme de fibres consistant en un mélange de gellane et d'un dérivé d'acide hyaluronique, contenant facultativement des substances biologiquement et/ou pharmacologiquement actives, obtenu selon les revendications 5 à 10.

13. Utilisation du biomatériau selon les revendications 1 à 4 et 12 pour la fabrication d'un médicament dans le traitement chirurgical de blessures de gravité légère à moyenne, telles que coupures, excoriations et abrasions profondes, brûlures légères.

14. Utilisation du biomatériau selon les revendications 1 à 4 et 12 pour la fabrication d'un médicament pour absorber du sérum et/ou des fluides biologiques au cours d'une chirurgie.
